Europäisches Patentamt

European Patent Office

Office européen des brevets

(11) Veröffentlichungsnummer: 0 067 314
A2

## (12) EUROPÄISCHE PATENTANMELDUNG

(21) Anmeldenummer: 82104170.4

(22) Anmeldetag: 13.05.82

(51) Int. Cl.³: **G 01 N 33/00**

(30) Priorität: 15.06.81 CH 3925/81

(43) Veröffentlichungstag der Anmeldung:
22.12.82 Patentblatt 82/51

(84) Benannte Vertragsstaaten:
AT CH DE FR GB LI NL

(71) Anmelder: CERBERUS AG
Alte Landstrasse 411
CH-8708 Männedorf(CH)

(72) Erfinder: Bukowiecki, Stanislaw, Dr.
Schwarzbachstrasse 30
CH-8713 Uerikon(CH)

(74) Vertreter: Tiemann, Ulrich, Dr.-Ing.
c/o Cerberus AG Patentbüro Alte Landstrasse 411
CH-8708 Männedorf(CH)

(54) Verfahren und Vorrichtung zur Bestimmung von Luftwechselzahlen.

(57) Zur Bestimmung der Luftwechselzahl in einem Raum wird als Tracergas ein reduzierend wirkendes Gas in dem Raum verteilt und die Konzentrationsabnahme des Gases mit einem bei Einwirkung reduzierender Gase seinen elektrischen Widerstand ändernden Gassensor gemessen. Als Tracergas kann vorzugsweise Propan verwendet werden, wobei die Konzentrationsabnahme mit einem nach dem Prinzip der katalytischen Oxidation arbeitenden Gassensor gemessen wird. Das Verfahren kann ohne aufwendige und teure Apparaturen durchgeführt werden und liefert schnell verlässliche Messwerte.

Fig.1

EP 0 067 314 A2

Verfahren und Vorrichtung zur Bestimmung von Luftwechselzahlen
_____

Die Erfindung geht aus einem Verfahren zur Bestimmung der
Luftwechselzahl der im Oberbegriff des Patentanspruchs 1 angegebenen Art.

In den letzten Jahren wurden vor allem in Klimazonen der Erde,
in denen die Gebäude mindestens ein Teil des Jahres hindurch
geheizt werden müssen, grosse Anstrengungen unternommen, den
Energieverbrauch für das Heizen zu reduzieren. Andererseits
ist es üblich geworden, in den tropischen Zonen der Erde Klimaanlagen in Wohn- und Arbeitsräumen zu installieren, welche
in den Räumen eine für den Menschen angenehme Temperatur aufrechterhalten. Der jährliche Endenergieverbrauch eines Gebäudes pro Bruttogeschossfläche ($m^2$) wird als Energiekennzahl E
bezeichnet und in Megajoule (MJ) per annum (a) angegeben.

$$E \quad [MJ/m^2 \cdot a]$$

Die Grösse der Energiekennzahl E wird von verschiedenen Faktoren bestimmt, beispielsweise vom Wirkungsgrad der Heizung
bzw. der Klimaanalge, vom Wärmeschutz des Gebäudes, d.h. den
Wärmedurchgang durch Wände, durch das Dach usw., von der
Strahlungsenergie, die durch Fenster und Glaswände abgegeben
bzw. aufgenommen wird, sowie durch den gewollten oder ungewollten Luftaustausch durch Lüftung oder Undichtigkeiten. Der
Energieverlust eines Gebäudes wird also vor allem durch drei
Faktoren beeinflusst, nämlich Wärmeleitung, Wärmestrahlung
und Wärmeströmung (Konvektion, Diffusion). Das Hauptaugenmerk
bei der Verringerung der Energiekennzahl wurde bislang auf
die Wärmeleitung gerichtet. Durch Verwendung von wärmedämmenden
Baumaterialien mit möglichst geringem Wärmedurchgangskoeffizienten k [$W/m^2 \cdot K$] konnten die Energieverluste durch Wärmeleitung

in gewünschtem Masse eingedämmt werden. Bei der Wärmestrahlung steht man in gewisser Weise vor einem Dilemma, da eine Verhinderung der Wärmeabstrahlung in den meisten Fällen gleichzeitig eine Verhinderung der Wärmeaufnahme bei Sonneneinstrahlung bedeutet.

In den letzten Jahren hat man in vermehrtem Masse das Augenmerk auf die Konvektionsverluste durch Lüftung und Undichtigkeiten gerichtet. Dabei ist es von Wichtigkeit zu wissen, innerhalb welchen Zeitraums die gesamte Luft in einem Gebäude durch die Aussenluft ersetzt wird oder anders ausgedrückt, welches Verhältnis zwischen dem einen Gebäude in einer Stunde zuströmenden Luftvolumen $V$ und seinem gesamten Raumvolumen $V_R$ besteht. Dies ist die sogenannte Luftwechselzahl n [$h^{-1}$].

$$n = \frac{V}{V_R}$$

Nach der SIA-Empfehlung 380 (abgedruckt im Sanierungshandbuch "Planung und Projektierung") ergeben sich je nach der Ausgesetztheit gegenüber dem Wind in Abhängigkeit von der Fensterdichtheit Luftwechselzahlen zwischen 0,3 und 2,0. Daraus wird ersichtlich, welche Energieverluste durch Undichtigkeiten hervorgerufen werden können und wie wichtig die genaue Kenntnis der Luftwechselzahl n bei gegebenen äusseren Bedingungen, z.B. Windströmung, Druckverhältnisse etc., ist.

Nach dem oben erwähnten Sanierungshandbuch erfolgt die Berechnung der Lüftungsverluste durch Entnahme der Luftwechselzahlen aus Tabellen, d.h. durch grobe Abschätzung. Von einer Messung der wirklich auftretenden Verluste kann bei diesem Verfahren nicht die Rede sein. Nach einem in der Zeitschrift "Energie", Nr. 2/81, angegebenen Verfahren wird in der Eingangstüre des Hauses oder in einem Fenster ein Ventilator angebracht, der es erlaubt, die den Fugen der Bauhülle entsprechende Fläche mit Hilfe eines Differentialdruckmessers zu ermitteln. Durch Herstellung eines Unterdrucks wird es möglich, das Eindringen von Kaltluft zu lokalisieren, und zwar entweder mit Hilfe einer Infrarot-Kamera oder mit einer Räucherpatrone. Diese Methode gestattet das Auffinden von Leckstellen, gibt aber keinen Aufschluss über die Luftwechselzahlen. Die bisher angewendeten experimentellen Bestimmungen der Luftwechselzahlen sind aufwendig und teuer. Beispielsweise wird Lachgas ($N_2O$) als Tracer in einem Raum verteilt und die Abnahme der Gaskonzentrationen mittels NDIR (nicht-dispersibles Infrarot-Gerät) gemessen und ausgewertet. Solche Geräte sind jedoch kompliziert in der Anwendung und ausserordentlich kostspielig.

Es besteht also weiterhin ein dringender Bedarf an einem Verfahren zur Bestimmung der Luftwechselzahl in Räumen, welches ohne aufwendige und teure Apparaturen anwendbar ist und möglichst schnell reale Messwerte liefert. Diese Aufgabe wird erfindungsgemäss durch die im kennzeichnenden Teil des Patentanspruchs 1 definierten Merkmale gelöst. Weitere zweckmässige und vorteilhafte Ausbildungen des erfindungsgemässen Verfahrens sind in den weiteren Ansprüchen erfasst.

Im folgenden wird anhand eines Ausführungsbeispiels das erfindungsgemässe Verfahren näher erläutert.

Fig. 1 stellt in schematischer Darstellung eine Anordnung zur Durchführung des erfindungsgemässen Verfahrens in einer Dreizimmerwohnung dar.

Fig. 2 zeigt den zeitlichen Verlauf der Konzentration an Tracergas in dem Raum, in welchem die Luftwechselzahl bestimmt werden soll.

Fig. 3 zeigt die verwendete Messanordnung.

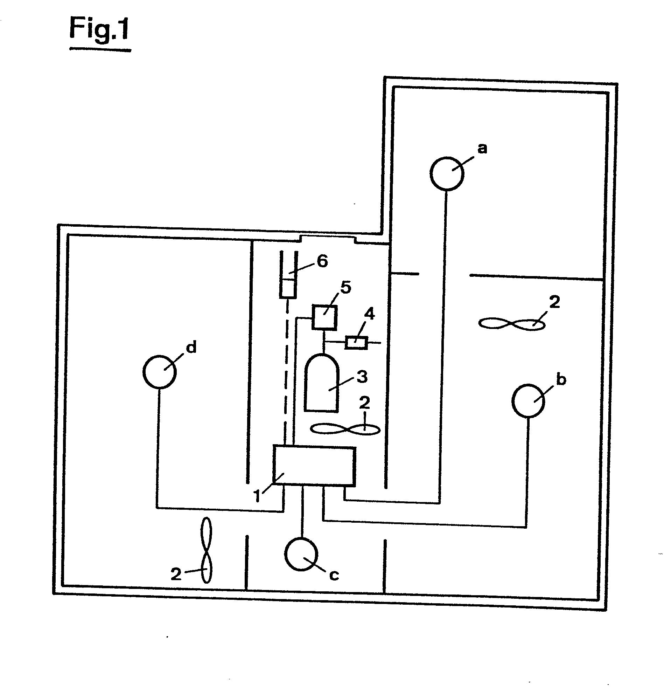

Die Messanordnung nach Fig. 1 besteht aus einem Steuergerät 1, welches die vier Gassensoren a, b, c, d speist und deren Signale auswertet, einem oder mehreren Ventilatoren 2, welche für die rasche Durchmischung von Tracergas und Luft sorgen, einer Trägergasvorratsflasche 3 und einem Gasströmungsmeter 4, mit welchem man die ausgeströmte Gasmenge bestimmen kann. Das Tracergasvorratsgefäss kann beispielsweise anstelle von einem manuellen mit einem elektrischen Gasventil 5 versehen sein, welches sich durch Steuersignale vom Steuergerät 1 betreiben lässt. Für einfache Räume ist es zweckmässig, nur einen Sensor a zu verwenden. Für zu untersuchende Räume, welche aus verschiedenen miteinander verbundenen Räumen bestehen, z.B. in Wohnungen, ist es vorteilhaft, mehrere Sensoren gleichzeitig zu verwenden, welche dann in den einzelnen Räumen plaziert werden. Das Beispiel in Fig. 1 erläutert diesen Punkt ausreichend.

Während der Durchführung der Bestimmung müssen in dem Raum, in dem die Luftwechselzahl bestimmt wird, annähernd konstante Bedingungen herrschen, d.h. es dürfen keine Fenster und/oder Türen geöffnet oder geschlossen werden. Wird die Verteilung des Tracergases in dem Raum mittels eines oder mehrerer Ventilatoren durchgeführt, so sollten die Ventilatoren auch während der Durchführung der Bestimmung laufen, um möglichst konstante Bedingungen zu erhalten.

An das Tracergas sind folgende Anforderungen zu stellen:

1.  nicht toxisch
2.  keine Explosions- bzw. Brandgefahr für die zur Messung notwendige Gaskonzentration

-5-

3. eindeutige Gassensor-Signale, grosse Ansprechempfindlichkeit und Dynamik
4. leicht erhältich und preiswert
5. leicht anwendbar
6. umweltfreundlich
7. schnell diffundierend
8. etwa gleiche Dichte wie Luft
9. keine Adsorption an Wänden, Textilien etc.


Für die praktische Anwendung ist die Verwendung von Propan
oder Propan/Butangemischen besonders vorteilhaft. Bei Verwendung von Alkoholdampf, welcher mit dem Verdampfergerät 6
erzeugt wird, besteht in gewissen Räumen die Gefahr, dass der
Dampf an den Wänden adsorbiert wird, was eine zu hohe Luftwechselzahl vortäuschen kann. Dies ist vor allem dann der Fall,
wenn sehr kleine Luftwechselzahlen gemessen werden müssen,
also solche, welche wesentlich unter $1.0 \text{ h}^{-1}$ liegen. Für genaue Bestimmungen von Luftwechselzahlen empfiehlt es sich,
verschiedene Gase als Tracergas zu verwenden. Mit dem gleichen
erfindungsgemässen Gerät kann dies durchgeführt werden, indem
die Messungen nacheinander mit verschiedenen Gasen, z.B. Propan und Alkoholdampf, durchgeführt werden. Das Gerät muss also
nicht verändert werden, was gegenüber den spektroskopischen
Methoden ein weiterer Vorteil ist.

Die folgende Beschreibung beschränkt sich auf Propan als Tracergas, welches sich besonders gut geeignet hat.Die untere
Explosionsgrenze von Propan liegt bei 2.1 Vol%. Die für eine
gute Messung notwendige Gaskonzentration liegt zwischen 500
- 1000 Vol.-ppm, was gegenüber der Explosionsgefahr eine mehr
als zehnfache Sicherheit ergibt. Ebenfalls liegt diese Konzentration unter der maximalen Arbeitskonzentration von 1000
Vol.-ppm, welcher ein Mensch während 8 Stunden ohne Schaden
ausgesetzt werden kann. (Die Bestimmung der Luftwechselzahl
dauert etwa 1-2 Stunden).

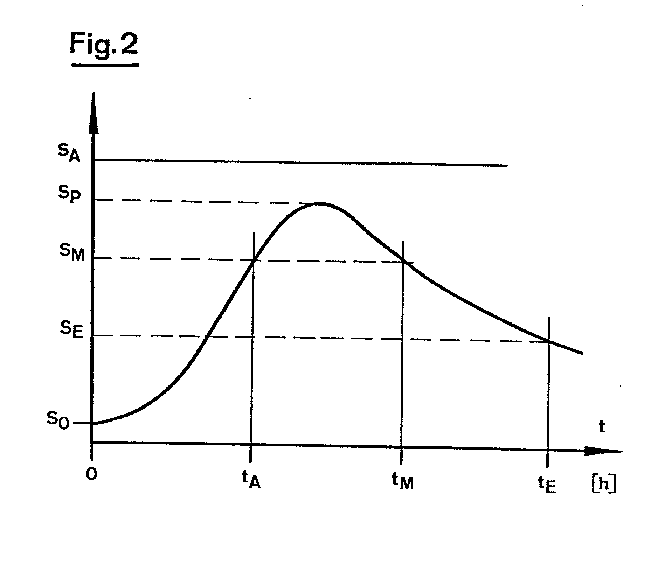

In Fig. 2 ist der zeitliche Verlauf der Konzentration des Tracergases (Propan) in einem Raum aufgezeichnet. Als Mass für die Konzentration dient die Signalstärke des Gassensors, welche als Ordinate aufgetragen wird. Die Signalstärke des Gassensors ist proportional zum elektrischen Leitwert des Metalloxids, z.B. Zinndioxid, welcher von der Gaskonzentration nach einer durch Gleichung (2) (siehe unten) beschriebenen Beziehung abhängig ist. Für Sensoren, welche auf dem Wärmetönungsprinzip beruhen, ist die Signalstärke proportional zur Wärmetönung, und damit proportional zur Tracergaskonzentration. Besonders vorteilhaft ist die Verwendung von Sensoren, welche die Aenderung des elektrischen Leitwertes von Metalloxiden zur Bestimmung der Gaskonzentration ausnützen.

In Beziehung zur Fig. 2 wird zur Zeit t = 0 die Gasflasche geöffnet. Die Ventilatoren 2 wurden schon früher eingeschaltet und sind während des ganzen Versuches in Betrieb. Die Signalstärke von beispielsweise Sensor c in Fig. 1 steigt rasch an. Mit kleinen Verzögerungen sprechen auch die in Fig. 1 in den entfernteren Räumen plazierten Sensoren a, b, d an. Nach einer gewissen Zeit $t_A$ steigt die Signalhöhe über einen bestimmten Wert $S_M$, welcher für eine Messung der Luftwechselzahl genügend hoch ist, womit das Ventil an der Gasflasche zugedreht werden kann. Die Zeit $t_A$ wird am besten vor dem Experiment aus dem Kubikinhalt des zu untersuchenden Raumes, der angestrebten Gaskonzentration (500 - 1000 Vol.-ppm) und der durch das Strömungsmeter 4 fliessenden Gasmenge errechnet. Es hat sich als vorteilhaft erwiesen, eine Strömungsmenge von etwa 5 Liter pro Minute zu wählen, so dass für einen Raum von 50 m³ Inhalt die Gasflasche etwa 10 Minuten lang geöffnet ist. Nach der Zeit $t_A$ erfolgt eine Wartezeit, bis die Gasmenge den Raum durch die Ventilatoren 2 homogen vermischt ist. Die Gaskonzentration beginnt dann abzufallen infolge von Diffusion nach aussen durch die Oeffnungen im Raum (Fensterfugen etc.). Der Abfall der Gaskonzentration erfolgt nach einem Exponentialgesetz

$$c(t) = c_O \exp(-nt) \qquad (1)$$

- 7.-

worin n die zu bestimmende Luftwechselzahl ist: $c_O$ die Gaskonzentration zum Zeitpunkt t = 0. Für den Zusammenhang zwischen der Gaskonzentration und dem elektrischen Leitwert für Metalloxidhalbleiter, wie z.B. Zinndioxid gilt

$$S = S_O + \beta c^\alpha \qquad (2)$$

worin $\alpha$ und $\beta$ Konstanten sind. Für genügend hohe Konzentrationen kann der Wert $S_O$ vernachlässigt werden. Damit berechnet sich die Luftwechselzahl aus den Gleichungen (1) und (2) nach der Formel

$$n = \frac{1}{\alpha \ (t_E - t_M)} \ \ln \left| \frac{(S_M - S_O)}{(S_E - S_O)} \right| \qquad (3)$$

Die in Gleichung (3) angegebenen Grössen können der Fig. 2 entnommen werden.

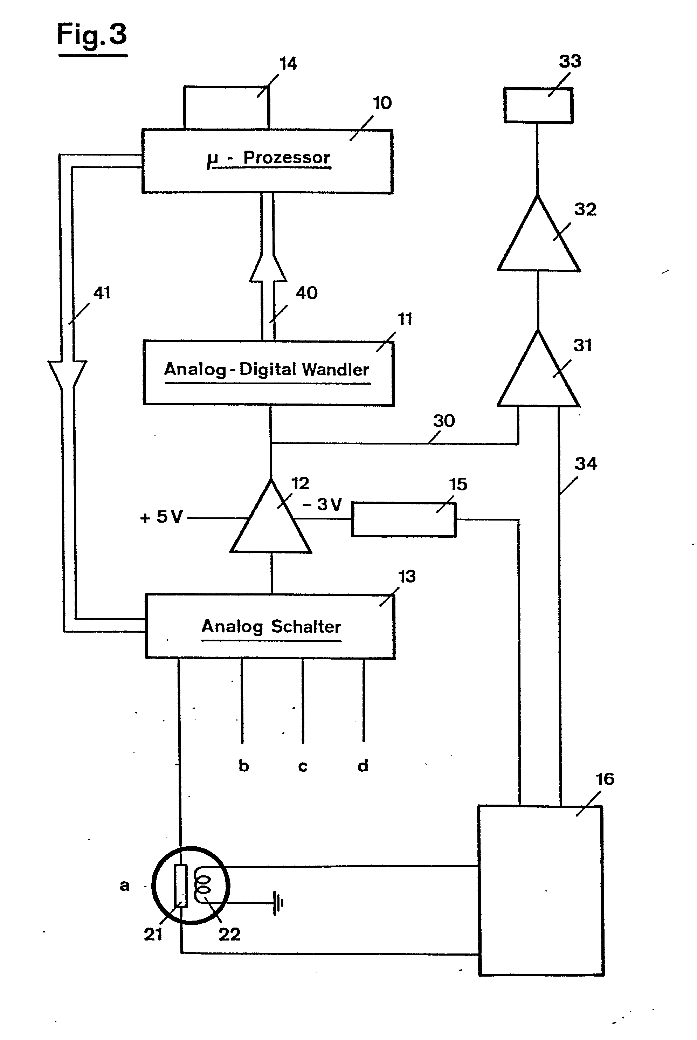

In Fig. 3 ist das verwendete Steuergerät in besserem Detail dargestellt. Die zentrale Einheit ist ein μ-Rechner 10, z.B. ein AIM 65 von Rockwell International. Die Sensoren a, b, c und d werden von einem Speisegerät 16 mit den notwendigen Spannungen an die Heizwiderstände 22, normalerweise 4-6 Volt, und den Metalloxidwiderstand 21, normalerweise 5 Volt, versorgt. Die Spannungssignale der Gassensoren a, b, c und d werden schrittweise vom über den Bus 41 gesteuerten Quad Analog Schalter 13 abgetastet und über den Verstärker 12 und den A/D-Wandler 11 und den Bus 40 dem Rechner 10 zugeführt. Der μ-Rechner 10 druckt in vorgegebenen Zeitabständen die Werte der Sensoren auf den Drucker 14 aus und berechnet nach bekannten Algorithmen laufend die Luftwechselzahl gemäss Gleichung (3). Die Sensorsignale werden weiter über die Leitung 30 einem Komparator 31 zugeführt, welcher das Signal mit einem Referenzsignal 34 vergleicht. Beim Ueberschreiten des Referenzsignals wird über die Verstärkerstufe 32 die Alarmstufe 33 aktiviert, die ein Warn- bzw. Alarmsignal abgibt. Dieser
Fall sollte nur durch extreme Unachtsamkeit des Experimentierers auftreten, da der Sicherheitsabstand von den explosiven Konzentrationen mehr als eine Grössenordnung beträqt.
Die vom Rechner ausgedruckten Werte der Luftwechselzahlen
für die vier im Beispiel angegebenen Sensoren müssen dann
vom Experimentierer nach seinem Ermessen interpretiert und
weiterverarbeitet werden. Der Spannungswandler 15 dient
zur Aufbereitung der für den Verstärker 12 notwendigen Spannung von -3 Volt.

Verfahren und Vorrichtung zur Bestimmung
von Luftwechselzahlen -
————————————————————————————————————————————

| Bezugszahlen | – | reference numbers |
|---|---|---|
| Steuergerät | 1 | control unit |
| Ventilator | 2 | ventilator |
| Trägergasvorratsflasche | 3 | tracergas tank |
| Strömungsmeter | 4 | flowmeter |
| Gaseinlassventil | 5 | gasinlet valve |
| Verdampfereinrichtung | 6 | evaporator |
| µ-Rechner | 10 | µ-computer |
| A/D-Wandler | 11 | A/D converter |
| Verstärker | 12 | amplifier |
| Quad Analog Schalter | 13 | quad analog switch |
| Drucker | 14 | printer |
| Spannungswandler | 15 | power supply |
| Speisegerät | 16 | power supply |
| Metalloxidwiderstand | 21 | metaloxide resistor |
| Heizwiderstand | 22 | heating coil |
| Leitung | 30 | lead |
| Komparator | 31 | comparator |
| Verstärkerstufe | 32 | amplifier |
| Alarmstufe | 33 | alarm stage |
| Referenzsignal | 34 | reference signal |
| Bus | 40 | bus |
| Bus | 41 | bus |
| Gassensor | a | gassensor |
| | b | |
| | c | |
| | d | |

Beiblatt zu C 229 00

Mögliche Spezifikationen der wichtigsten Elemente der
Schaltung Fig. 3, C 229 00.

| 10/14 | μ-Prozessor mit Drucker | AIM 65 Rockwell International |
|---|---|---|
| 11 | Analog/Digital-Wandler | ADC 0801 |
| 12 | Verstärker op-amp | ICL 7611 |
| 13 | Quad Analog Schalter | 4066 |
| 15 | Spannungswandler | 4049 |
| a,b,c,d | Gassensoren | TGS 812 |

Patentansprüche
_____

1. Verfahren zur Bestimmung der Luftwechselzahl in einem Raum durch Verteilung eines Tracergases in dem Raum und Bestimmung der zeitabhängigen Gaskonzentrationsabnahme, dadurch gekennzeichnet, dass als Tracergas ein reduzierend wirkendes Gas verwendet wird und dass die Konzentrationsabnahme mit einem bei Einwirkung reduzierender Gase seinen elektrischen Widerstand ändernden Gassensor gemessen wird.

2. Verfahren gemäss Patentanspruch 1, dadurch gekennzeichnet, dass der Gassensor ein Halbleiter ist, der in einem Temperaturbereich von 200 bis 400°C bei Einwirkung reduzierender Gase seinen Widerstand ändert.

3. Verfahren gemäss Patentanspruch 1, dadurch gekennzeichnet, dass der Gassensor ein nach dem Prinzip der katalytischen Oxidation arbeitender Gassensor ist.

4. Verfahren gemäss einem der Patentansprüche 1 bis 3, dadurch gekennzeichnet, dass als Tracergas Alkoholdampf verwendet wird.

5. Verfahren gemäss einem der Patentansprüche 1 bis 3, dadurch gekennzeichnet, dass als Tracergas Propan oder ein Gemisch von Propan mit Butan verwendet wird.

6.  Vorrichtung zur Durchführung des Verfahrens gemäss einem
    der Patentansprüche 1 bis 3, gekennzeichnet durch mindestens einen bei Einwirkung von reduzierenden Gasen seinen elektrischen Widerstand ändernden Gassensor (a, b, c, d),
    ein Steuergerät (1), Ventilatoren (2), eine Tracergas-Erzeugungs-Vorrichtung (3, 4, 5) die vom Steuergerät (1) betätigbar ist, eine Anzeigevorrichtung (14) für die Gaskonzentration und eine Alarmvorrichtung (31, 32, 33) zur Anzeige von gefährlichen Gas-Konzentrationen.

7.  Vorrichtung gemäss Patentanspruch 6, dadurch gekennzeichnet,
    dass die Tracergas-Erzeugungs-Vorrichtung aus einem Tracergas-Vorratsgefäss (3), einem Gasventil (5), das so ausgebildet ist, dass es vom Steuergerät (1) betätigbar ist und einem Gasströmungsmeter (4) besteht.

8.  Vorrichtung zur Durchführung des Verfahrens gemäss Patentanspruch 6, dadurch gekennzeichnet, dass die Tracergas-Erzeugungs-Vorrichtung aus einem mit Alkohol gefüllten, beheizbaren Behälter (6) besteht, mit welchem das Tracergas
    Alkohol durch Verdampfen erzeugt wird, wobei der Verdampfungsprozess durch das Steuergerät (1) regelbar ist.

9.  Vorrichtung, gemäss einem der Patentansprüche 6 bis 8, dadurch gekennzeichnet, dass das Steuergerät (1) einen μ-Rech-
    ner (10), einen Quad-Analog Schalter (13), mit welchem die
    Signale der Gassensoren (a, b, c, d) abgetastet und über
    einen ersten Verstärker (12) und einen Analog/Digital-Wand-
    ler (11) dem μ-Rechner (10) zugeführt werden, und einen Komparator (31), der bei Ueberschreiten der Sensorsignale
    über ein Referenzsignal (34) über einen weiteren Verstärker
    (32) eine Alarmstufe (33) ansteuert, aufweist, und dass die
    Anzeigevorrichtung (14) als Druckvorrichtung ausgebildet
    ist.

10. Vorrichtung gemäss einem der Patentansprüche 1 bis 9, dadurch gekennzeichnet, dass als Gassensoren (a, b, c, d) Wärmeleitungssensoren verwendet werden.

11. Vorrichtung gemäss Patentansprüchen 1 bis 9, dadurch gekennzeichnet, dass als Gassensoren (a, b, c, d) katalytische Wärmetönungssensoren verwendet werden.

## Fig.1

## Fig.2

## Fig.3